# FASCICULE DE BREVET EUROPEEN

(11) **EP 4 045 141 B1**
(45) Date de publication et mention de la délivrance du brevet: **05.03.2025**
(21) Numéro de dépôt: 20803222.7
(22) Date de dépôt: 16.10.2020
(51) Int. Cl.: A61N 5/10

(54) **SYTÈME DE SUIVI D'UN FAISCEAU D'HADRONS PENDANT UN TRAITEMENT D'HADRONTHERAPIE D'UN SUJET**
SYSTEM ZUR ÜBERWACHUNG EINES HADRONSTRAHLS WÄHREND DER HADRONTHERAPIEBEHANDLUNG EINER PERSON
SYSTEM FOR MONITORING A HADRON BEAM DURING HADRON-THERAPY TREATMENT OF A SUBJECT

(30) Priorité: 18.10.2019 FR 1911704
(43) Date de publication de la demande: 24.08.2022
(73) Titulaire: Centre national de la recherche scientifique, 75016 Paris (FR); Institut Mines Télécom, 91120 Palaiseau (FR)
(72) Inventeur: THERS, Dominique, 44119 Treillières (FR); STUTZMANN, Jean-Sébastien, 44700 Orvault (FR)
(74) Mandataire: Cabinet Nony
(86) Numéro de dépôt international: PCT/FR2020/051863
(87) Numéro de publication internationale: WO 2021/074551

(56) Documents cités:
- EP-A1- 3 305 200
- WO-A1-2013/036811
- WO-A1-2017/156113
- GALLEGO MANZANO L ET AL: "XEMIS: A liquid xenon detector for medical imaging", NUCLEAR INSTRUMENTS & METHODS IN PHYSICS RESEARCH. SECTION A, vol. 787, 20 November 2014 (2014-11-20), pages 89 - 93, XP029157774, ISSN: 0168-9002, DOI: 10.1016/J.NIMA.2014.11.040
- PARODI KATIA ED - UNNO YOSHINOBU ET AL: "On- and off-line monitoring of ion beam treatment", NUCLEAR INSTRUMENTS & METHODS IN PHYSICS RESEARCH. SECTION A, vol. 809, 6 July 2015 (2015-07-06), pages 113 - 119, XP029372104, ISSN: 0168-9002, DOI: 10.1016/J.NIMA.2015.06.056

## Description

### DOMAINE DE L'INVENTION

L'invention a trait à un système de suivi d'un faisceau d'hadrons pendant un traitement d'hadronthérapie d'un sujet atteint de cancer. En particulier, l'invention a trait à un système de suivi de la zone d'interaction entre les hadrons du faisceau et les tissus du sujet, et donc indirectement de la dose délivrée, par rapport à la masse tumorale à traiter qui a été marquée avec un produit radiopharmaceutique comprenant un radio-isotope émettant un positron et un rayon gamma de désexcitation en quasi-coïncidence.

### ÉTAT DE LA TECHNIQUE

L'hadronthérapie est un traitement du cancer hautement efficace qui utilise des faisceaux de particules de protons ou d'ions carbone. Cette technique innovante a prouvé son efficacité dans le cas des tumeurs difficiles à traiter par voie de radiothérapie conventionnelle car elles sont soit radio-résistants, soit situées en profondeur ou proches des organes vitaux. L'utilisation de faisceaux de hadrons permet une grande précision dans le dépôt de la dose, à la fois longitudinalement et transversalement. En effet, contrairement aux faisceaux de photons dont le dépôt d'énergie est maximal dès les premiers centimètres à l'entrée du patient et s'atténue avec la profondeur, les ions chargés déposent leurs maximums d'énergie à la fin de leurs parcours (pic de Bragg) tout en maintenant une dose minimale déposée en entrée. En outre, la pénombre latérale des protons est plus faible que celle obtenue avec un faisceau de photons. Toutefois, la haute précision balistique de l'hadronthérapie fait de que cette technique soit sensible à toute source de déviation par rapport au plan de traitement : mauvais positionnement du patient, mouvements d'organes ou changements anatomiques entre les fractions ou encore régression de la tumeur, perte de poids, remplissage de cavité anatomique entre les séances de traitement.

Afin d'exploiter l'avantage de la haute précision balistique, il est nécessaire un contrôle rigoureux de la localisation de la dose délivrée par rayonnement. Lorsque le faisceau primaire s'arrête à l'intérieur du patient, le suivi en temps réel du parcours des hadrons peut être réalisé au moyen de radiations secondaires, notamment de rayons gamma, issus de réactions nucléaires des hadrons dans le corps.

Certaines modalités d'imagerie apparaissent prometteuses pour obtenir des informations concernant le profil du faisceau d'hadron, tels que les ultrasons induits par les ions, la mesure d'électrons secondaires de bremsstrahlung ou l'imagerie par résonance magnétique (IRM) qui présente des problèmes spécifiques associés à la déflexion du faisceau d'hadrons dans le champ magnétique en cas d'IRM en temps réel. Cependant, ces techniques peuvent être difficiles à réaliser. Actuellement, des systèmes de tomographie par émission de positons (TEP) « in-beam » ont été proposés qui exploitent les rayons gamma coïncident à 511 keV issus de l'annihilation de positions émis au cours de la désintégration beta des isotopes radioactifs générés par l'interactions des hadrons dans les tissus. Cependant, à cause du faible nombre d'isotopes subissant une désintégration bêta, et du fait que l'observation de la désintégration bêta est retardée, par rapport à la désintégration, de la durée du temps de vie des isotopes radioactifs, il est nécessaire un temps d'acquisition de données assez long. En conséquence, l'imagerie du faisceau en temps réel avec la TEP in-beam donne seulement une information concernant la position de la dose délivrée après le traitement. De plus, contrairement au system TEP conventionnel, le système TEP in-beam a un angle de couverture réduit pour permettre le positionnement de la tête rotative iso-centrique « Gantry », ce qui réduit le volume sensible du système.

Dans ce contexte, Frandes et al. (Frandes, Mirela, et al. "A tracking Compton-scattering imaging system for hadron therapy monitoring." IEEE Transactions on Nuclear Science 57.1 (2010): 144-150.) ont démontré, par simulations numériques, la faisabilité d'un système pour l'imagerie médicale basé sur la combinaison d'un télescope Compton et d'une caméra de création de paires. Les simulations du système pour des différentes énergies du faisceau, typique de l'hadronthérapie, ont permis à Frandes et al. de démontrer la capacité de ce système d'imagerie à détecter des rayons gamma dans un régime d'énergie caractéristique des interactions Compton de sorte à reconstruire la position de la dose délivrée. Toutefois, ce système visant à la détection de rayons gamma issus de l'annihilation, présente le même inconvénient que le in-beam TEP.

Déplus, l'anatomie interne du patient peut varier entre une séance et la suivante ou pendant une même séance. En conséquence, pouvoir suivre la position et la quantité de dose déposée dans le volume du patient en temps réel n'est pas suffisant pour garantir que le volume tumoral cible soit irradié dans sa totalité tout en minimisant le plus possible la dose délivrée aux tissus sains dans les alentours. En effet, un déplacement du volume tumoral par rapport aux repères anatomiques externes qui sont utilisés pour aligner le patient par rapport au faisceau peut avoir de lourdes conséquences du fait que la dose ne sera pas délivrée selon le plan de traitement et donc des tissus sains seront irradiés. C'est à ces inconvénients qu'entend plus particulièrement remédier l'invention en proposant un système permettant de suivre en temps réel pendant le traitement d'hadronthérapie, à la fois la position de la masse tumorale et la position de la dose délivrée par le faisceau dans le patient permettant un dépôt de la dose conforme au plan de traitement.

### RÉSUMÉ

L'invention est définie dans les revendications attenantes. Un procédé de suivi d'un faisceau d'hadrons pendant un traitement d'hadronthérapie d'un sujet est décrit mais ne fait pas partie de l'objet revendiqué. Il est décrit à titre illustratif un procédé de suivi d'un faisceau d'hadrons pendant un traitement d'hadronthérapie d'un sujet comprenant des cellules tumorales marquées avec un produit radiopharmaceutique ayant un radio-isotope émettant un positron et un rayon gamma de désexcitation en quasi-coïncidence, dans lequel le traitement d'hadronthérapie est délivré selon un plan de traitement comprenant des paramètres prédéfinis en fonction du temps afin de définir au moins une caractéristique du faisceau d'hadrons dans le temps pendant le traitement d'hadronthérapie, dans lequel le faisceau d'hadrons comprend une pluralité de « burst »s discrets d'hadrons émis à une fréquence prédéfinie par un dispositif d'accélération, le procédé comprenant les étapes suivantes :
- quand un « burst » impacte le sujet :
   - détecter les « gamma prompts » générés par l'interaction des hadrons du « burst » avec les tissus du sujet à l'aide d'un télescope Compton au xénon liquide ;
   - utiliser les « gamma prompts » détectés pour reconstruire une image du volume d'interaction à l'intérieur duquel les hadrons du « burst » interagissent avec les tissus du sujet ;
- quand aucun « burst » n'impacte le sujet :
   - extraire la position des cellules tumorales marquées avec le produit radiopharmaceutique en détectant simultanément le rayon gamma de désexcitation et deux rayons gamma d'annihilation produits par le positron à l'aide du télescope Compton au xénon liquide ;
   - reconstruire une image du volume total de la tumeur à traiter par le faisceau d'hadrons pendant le traitement d'hadronthérapie ;
- comparer l'image du volume d'interaction et l'image du volume total de la tumeur de sorte à localiser le volume d'interaction mesuré par rapport au volume total mesuré de la tumeur ;
- à chaque fois que le volume d'interaction mesuré est compris au moins partiellement dans le volume total mesuré de la tumeur, calculer l'écart entre, d'une part, la position du volume d'interaction mesuré dans le volume total mesuré de la tumeur et, d'autre part, une position prédéfinie du volume d'interaction dans le volume total de la tumeur définie dans le plan de traitement.

Grâce à l'invention, il est possible de suivre en temps réel une séance de traitement d'hadronthérapie et évaluer de manière efficace si le traitement a été administré conformément au plan de traitement grâce à la reconstruction et comparaison de l'image du volume d'interaction et l'image du volume total de la tumeur.

Dans un mode de réalisation, l'image du volume d'interaction est obtenue pour chaque « burst » impactant le sujet. Cela permet de suivre en temps réel la position du faisceau dans le sujet.

Dans un mode de réalisation, le procédé comprenant en outre une étape dans laquelle l'écart entre la position prédéfinie du volume d'interaction et la position du volume d'interaction mesurée par rapport au volume total mesuré de la tumeur est comparée à un seuil prédéfini. De manière avantageuse, ce mode de réalisation permet de vérifier en temps réel que la distribution de la dose délivrée par le faisceau dans le sujet est conforme, dans une marge d'erreur acceptable d'un point de vue clinique, au plan de traitement.

Dans un mode de réalisation, à chaque fois que l'écart est supérieur au seuil prédéfini, le procédé comprend une étape de calcul d'au moins un nouveau paramètre du faisceau d'hadrons de manière à corriger au moins une caractéristique du faisceau d'hadrons. Un plan de traitement de radiothérapie est habituellement établi sur la base d'une image tridimensionnelle du sujet obtenue par tomographie axiale calculée par ordinateur « CT-scan » en convertissant les unités Hounsfield (HUs) en pouvoir d'arrêt des protons pour pouvoir calculer la distribution de la dose. Mais le CT-scan n'est qu'une image statique du sujet dans une position donnée et à un temps donné précédant au traitement. Toutefois en hadronthérapie, les mouvements des organes, comme les mouvements intestinaux, la respiration, les pulsations cardiaques ou le changement de l'anatomie interne dû au remplissage de la vessie ou une variation de poids, entraînent également des changements de densité, et donc une modification de la longueur du trajet radiologique, le long de la trajectoire du faisceau. Leur influence en protonthérapie peut entraîner un grave sous-dosage du volume clinique cible et un surdosage des organes à risques et des tissus normaux distaux de la cible. Ce mode de réalisation permet de prendre en compte tous changement de géométrie et de corriger les paramètres du faisceau d'hadrons en temps réel pour permettre de délivrer la dose dans le volume tumoral cible dans le plan de traitement.

Dans un mode de réalisation, le sujet est positionné sur un support mécanique motorisé configuré pour déplacer le sujet par rapport au faisceau pendant le traitement d'hadronthérapie. Dans ce mode de réalisation, au moins un de paramètres prédéfinis du plan de traitement correspond à la position dans l'espace du support mécanique motorisé.

Dans un mode de réalisation, à chaque fois que l'écart est supérieur au seuil prédéfini, le procédé comprend une étape de calcul du paramètre du plan de traitement correspondant à une nouvelle position dans l'espace du support mécanique motorisé de manière à corriger au moins une caractéristique du faisceau d'hadrons. Cela permet d'avoir la source du faisceau dans une position fixe et de s'affranchir de l'utilisation d'un « Gantry ».

Dans un mode de réalisation, le nouveau paramètre du faisceau d'hadrons est transmis au dispositif d'accélération, qui modifie au moins une caractéristique du faisceau d'hadrons de manière à modifier la position du volume d'interaction par rapport au volume total mesuré de la tumeur. Ceci crée une bouche de feedback permettant un ajustement en temps réel du faisceau au plan de traitement.

Dans un mode de réalisation, à chaque fois que l'écart est supérieur au seuil prédéfini, le procédé comprend l'arrêt du faisceau d'hadrons. De manière avantageuse, ce mode de réalisation permet d'arrêter le traitement pour éviter un surdosage des organes à risques. Dans un mode de réalisation, le procédé comprend en outre une étape de reconstruction d'une séquence d'image tridimensionnelle résultant de la fusion d'une image tridimensionnelle du volume d'interaction avec une image tridimensionnelle du volume total de la tumeur dans un même référentielle de la caméra Compton.

L'invention a pour objet un système de suivi d'un faisceau d'hadrons pendant un traitement d'hadronthérapie d'un sujet comprenant des cellules tumorales marquées avec un produit radiopharmaceutique ayant un radio-isotope émettant un positron et un rayon gamma de désexcitation en quasi-coïncidence, dans lequel le traitement d'hadronthérapie est délivré selon un plan de traitement comprenant des paramètres prédéfinis en fonction du temps afin de définir au moins une caractéristique du faisceau d'hadrons dans le temps pendant le traitement d'hadronthérapie, dans lequel le faisceau d'hadrons comprend une pluralité de « burst »s discrets d'hadrons émis à une fréquence prédéfinie par un dispositif d'accélération, le système comprenant :
- un module d'imagerie de faisceau configuré pour recevoir les données acquises d'un télescope Compton à xénon liquide lorsqu'un « burst » impacte le sujet et pour analyser ces données de manière à déterminer le point d'émission des « gamma prompts » générés par l'interaction du « burst » avec les tissus du sujet à partir duquel est reconstruite une image du volume d'interaction à l'intérieur duquel les hadrons du « burst » interagissent avec les tissus ;
- un module d'imagerie tumorale configuré pour recevoir les données acquises par le télescope Compton à xénon liquide lorsqu'aucun « burst » n'impacte le sujet et pour analyser ces données de manière à extraire la position des cellules tumorales marquées avec le produit radiopharmaceutique en détectant simultanément le rayon gamma de désexcitation et deux rayons gamma d'annihilation produits par le positron et utiliser la position des cellules tumorales pour reconstruire une image du volume total de la tumeur à traiter par le faisceau d'hadrons pendant le traitement d'hadronthérapie ;
- un module d'évaluation configuré pour comparer l'image du volume d'interaction et l'image du volume total de la tumeur de sorte à localiser le volume d'interaction mesuré par rapport au volume total mesuré de la tumeur et, à chaque fois que le volume d'interaction mesuré est compris au moins partiellement dans le volume total mesuré de la tumeur, pour calculer l'écart entre, d'une part, la position du volume d'interaction mesuré par rapport au volume total mesuré de la tumeur et, d'autre part, une position prédéfinie du volume d'interaction au sein du volume total de la tumeur définie dans le plan de traitement.

Le système mettant en œuvre le procédé décrit permet avantageusement de suivre en temps réel la dose délivrée dans le sujet grâce à la reconstruction de l'image du volume d'interaction et l'image du volume total de la tumeur, et de vérifier que celle-ci est bien conformée à la distribution de dose prévue dans le plan de traitement.

Dans un mode de réalisation, dans le module d'évaluation l'image du volume d'interaction est obtenue pour chaque burst impactant le sujet.

Dans un mode de réalisation, le module d'évaluation est en outre configuré pour comparer l'écart entre la position prédéfinie du volume d'interaction et la position du volume d'interaction mesuré par rapport au volume total mesuré de la tumeur, à un seuil prédéfini. Dans un mode de réalisation, le sujet est positionné sur un support mécanique motorisé configuré pour déplacer le sujet par rapport au faisceau pendant le traitement d'hadronthérapie et dans lequel au moins un des paramètres prédéfinis du plan de traitement correspond à la position dans l'espace du support mécanique motorisé. Selon un mode de réalisation, le système comprend en outre un module de correction configuré pour calculer au moins un nouveau paramètre du faisceau d'hadrons, à chaque fois que l'écart est supérieur au seuil prédéfini, afin de corriger au moins une caractéristique du faisceau d'hadrons.

Selon une caractéristique avantageuse, le module de correction est en outre configuré pour transmettre le nouveau paramètre du faisceau d'hadrons au dispositif d'accélération, qui modifie au moins une caractéristique du faisceau d'hadrons de manière à modifier la position du volume d'interaction par rapport au volume total mesuré de la tumeur. Selon un mode de réalisation, le système comprend en outre un module de sécurité configuré pour transmettre au dispositif d'accélération l'instruction d'arrêter le faisceau d'hadrons à chaque fois que l'écart est supérieur au seuil prédéfini.

Selon un mode de réalisation, le système comprend en outre un module de reconstruction d'image configuré pour reconstruire une séquence d'image tridimensionnelle résultant de la fusion d'une image tridimensionnelle du volume d'interaction avec une image tridimensionnelle du volume total de la tumeur selon un même référentiel de la caméra Compton.

Selon un mode de réalisation, le système comprend le télescope Compton, étant un télescope Compton au xénon liquide.

Selon un mode de réalisation, le système comprend le dispositif d'accélération et / ou le support mécanique motorisé.

Selon un mode de réalisation, le procédé de suivi d'un faisceau d'hadrons est un procédé mis en œuvre par ordinateur.

Il est décrit également un programme d'ordinateur comprenant des instructions qui, lorsque le programme est exécuté par un ordinateur, conduisent celui-ci à mettre en œuvre le procédé de suivi d'un faisceau d'hadrons décrit ci-dessus.

Il est décrit également un support d'enregistrement lisible par ordinateur comprenant des instructions qui, lorsqu'elles sont exécutées par un ordinateur, conduisent celui-ci à mettre en œuvre le procédé de suivi d'un faisceau d'hadrons décrit ci-dessus.

### DÉFINITIONS

Dans la présente invention, les termes ci-dessous sont définis de la manière suivante :
- « **Burst** » fait référence à un paquet d'hadrons ayant une même phase d'accélération.
- « **Gamma Prompts** » fait référence à des rayons gamma rapide et d'haute énergie qui sont naturellement émit à la suite de réactions nucléaires des hadrons avec les tissus du sujet.
- « **Organes A Risque (OAR)** » fait référence aux organes sur lesquels le médecin impose des contraintes de dose à ne pas dépasser afin d'éviter au mieux les effets secondaires liés à l'irradiation.
- « **PTV (ou Planning Target Volume)** », fait référence à un volume, qui inclut le la tumeur visible sur les examens d'imagerie (GTV ou « Gross Tumor Volume ») élargie de marges thérapeutiques définies par le médecin selon le type de pathologie traitée (CTV ou « Clinical Target Volume »), en y ajoutant une marge définie par les imprécisions dues au processus du traitement (incertitudes de parcours, de positionnement, incertitudes intrinsèques à la machine de traitement, etc.).
- « **Sujet** » fait référence à un mammifère, de préférence un être humain. Au sens de la présente invention, un sujet peut être un patient, c'est-à-dire une personne sous surveillance médicale, subissant ou ayant subi un traitement médical, ou faisant l'objet d'un suivi pour le développement d'une maladie.

### DESCRIPTION DÉTAILLÉE

Les caractéristiques et avantages de l'invention apparaîtront dans la description qui va suivre de plusieurs modes de réalisation d'un système selon l'invention, donnée uniquement à titre d'exemple et faite en se référant aux dessins annexés dans lesquels :
la **Figure 1** est une vue schématique en perspective d'une table de traitement et du télescope Compton en configuration ouverte, conformément à un premier mode de réalisation de l'invention ;
la **Figure 2** est un organigramme représentant les étapes du procédé selon un premier mode de réalisation ;
la **Figure 3** est une vue schématique en perspective d'une table de traitement et un télescope Compton en configuration ouverte, conformément à un deuxième mode de réalisation de l'invention ;
la **Figure 4** est une vue schématique d'une table de traitement et un télescope Compton, en configuration fermée, conformément à un troisième mode de réalisation de l'invention.

La présente invention concerne un système de suivi d'un faisceau d'hadrons pendant un traitement d'hadronthérapie d'un sujet atteint de tumeur.

Typiquement, dans un centre d'hadron thérapie les hadrons sont accélérés à des énergies thérapeutiques, comprises entre 70 et 250 MeV, généralement avec un cyclotron ou un synchrotron, et sont transportés dans la salle de traitement où ils pénètrent dans un nez de traitement. Les minces faisceaux initiaux d'hadrons sont répartis latéralement et longitudinalement et mis en forme de manière appropriée pour permettre des traitements. Les hadrons utilisés sont typiquement les protons ou les ions Carbon. La diffusion et la mise en forme du faisceau peuvent être réalisées par des moyens électromécaniques pour traiter les patients avec une hadronthérapie par diffusion passive (PSHT ou « passively-scattered hadron therapy »). Cette technique est notamment utilisée avec le proton. Un deuxième mode, appelé balayage uniforme, consiste à balayer un faisceau grossier sur un champ large qui est ensuite collimaté et compensé en profondeur par des accessoires personnalisés pour l'anatomie du patient. Cela permet de traiter des champs plus larges avec des parcours plus profonds. Une autre approche consiste dans le balayage magnétique de mini-faisceaux d'hadrons à une séquence d'énergies initiales. Les hadrons sont balayés très finement et précisément dans la tumeur dans les trois directions (x,y,z) afin d'irradier cette dernière sans avoir recours à des accessoires personnalisés. Cette dernière technique peut être utilisée pour traiter des patients avec une hadronthérapie à modulation d'intensité optimisée (« intensity modulated hadron therapy »).

Ledit traitement d'hadronthérapie est délivré selon un plan de traitement comprenant des paramètres prédéfinis en fonction du temps afin de définir au moins une caractéristique du faisceau d'hadrons dans le temps pendant le traitement d'hadronthérapie.

Selon un mode de réalisation, les paramètres prédéfinis que définissent au moins une caractéristique du faisceau varie selon la technique de hadronthérapie utilisée.

Pour la méthode de diffusion passive, la diffusion latérale et longitudinale du faisceau est obtenue grâce à l'utilisation d'une roue de modulation de parcours et un ou deux diffuseurs fait de matériaux à haute Z pour produire un large faisceau plat dans la région d'intérêt. La roue de modulation est configurée pour interposer de pâles de différentes épaisseurs de matériau sur le trajet des hadrons lors de sa rotation pour étaler le pic de Bragg et l'ajuster à la profondeur du volume cible. La modulation du pic de Bragg est donc discrète. Les épaisseurs et les largeurs de pâles sont conçues de manière à ce que la somme des pics de Bragg individuels résultants donne une distribution de doses de profondeur uniforme et homogène, le SOBP ou « spread-out Bragg peak ». Pour adapter la distribution de la dose latéralement à la forme du volume cible (plus des marges appropriées), une ouverture, généralement constituée de blocs de laiton d'épaisseur suffisante (2 cm à 8 cm) pour absorber les hadrons incidents de la plus haute énergie, est utilisée. Finalement, pour créer une distribution de dose conforme à la forme distale de la cible, le pic de Bragg du faisceau diffusé de manière passive est étalé plus loin à l'aide d'un compensateur de distance.

Selon un mode de réalisation, le traitement d'hadron thérapie utilise la technique de diffusion passive. Dans ce mode de réalisation, les paramètres prédéfinis comprennent au moins l'énergie du faisceau et la modulation du trajet des hadrons (i.e. SOBP). En effet, l'absorbeur et le compensateur sont des accessoires personnalisés pour le patient et leur position ne change pas pendant le traitement.

Pour le balayage uniforme, est utilisé un système de balayage actif qui, avec de multiples mini-irradiations, construit un pic de Bragg modulé dans la tumeur. L'irradiation du volume cible s'effectue par un balayage latéral d'une couche après l'autre. Une roue de modulation dédiée est utilisée pour translater les pics de Bragg pas à pas. Pour une meilleure définition du pic de Bragg modulé, des couches en profondeur intermédiaires sont irradiées grâce à l'association des plaques d'absorbeur d'un diffuseur et des pâles de la roue de modulation. Une fois modulé, le faisceau de protons est ensuite largement balayé dans les directions Y et X respectivement par un aimant vertical en première position puis par un aimant horizontal qui suit.

Selon un mode de réalisation, le traitement d'hadron thérapie utilise la technique de balayage uniforme. Dans ce mode de réalisation, les paramètres prédéfinis comprennent au moins l'énergie du faisceau, la modulation du trajet des hadrons et la position en X et Y du faisceau.

Le balayage actif de mini-faisceaux consiste en balayer un faisceau élémentaire d'ions précisément avec des aimants horizontalement et verticalement tout en changeant l'énergie initiale des protons afin de balayer couche par couche tout le volume tumoral sans avoir recours aux accessoires personnalisés du patient. La profondeur (Z) est gérée par la modulation d'énergie du faisceau de protons grâce au système de sélection d'énergie. La position du fin pinceau dans le plan (X, Y) est contrôlée grâce aux mêmes aimants de balayage que décrit ci-dessous. Un ensemble de spots est ainsi créé et distribué sur l'ensemble du volume cible. La taille du spot à l'iso-centre est adaptée grâce aux quadripôles situés avant les aimants de balayage. Son principal avantage est de diminuer la quantité de neutrons produits et la perte de débit, toutes deux dues aux accessoires de mise en forme passive du faisceau.

Selon un mode de réalisation, le traitement d'hadron thérapie utilise la technique de balayage actif de mini-faisceaux. Dans ce mode de réalisation, le paramètre prédéfini comprend au moins le coordonnées X, Y et Z (i.e. l'énergie du faisceau) et l'intensité de chaque spot.

Les techniques implémentant le balayage magnétique du faisceau peuvent être configurées pour permettre une déflexion du faisceau permettant de couvrir un champ de traitement pouvant atteindre 30 cm x 40 cm.

Dans une salle de traitement d'hadronthérapie, le sujet est normalement assis sur une chaise ou allongé sur une table de traitement dans une position prédéfinie par rapport au nez de traitement, duquel sort le faisceau pour impacter le sujet.

Dans un mode de réalisation, le nez de traitement se trouve dans une position fixe à une distance prédéfinie du sujet. Le nez de traitement peut être positionné par rapport au sujet de sorte à ce que la direction d'incidence du faisceau F rentre dans le sujet perpendiculairement au plan sagittal du sujet. Dans un mode de réalisation préféré, le nez de traitement est positionné face au sujet de sorte à ce que le faisceau ait son point d'entrée sur la partie antérieure du sujet. Cela permet au faisceau de pénétrer dans le sujet jusqu'au volume cible en traversant une épaisseur de tissus sains et/ou organes à risques plus faible possible, de sorte à réduire la dose qu'ils reçoivent.

Avec un nez de traitement en position fixe, la direction d'incidence du faisceau F, et donc aussi son point d'entrée dans les tissus, peut être variée seulement en utilisant le balayage magnétique, qui permet des déviations du faisceau de faible ampleur.

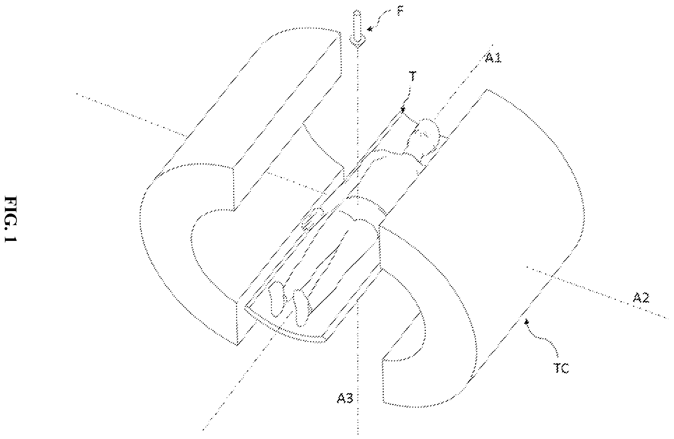

Pour permettre de varier l'angle d'incidence du faisceau sur un arc de plus que 180 degrés, dans un mode réalisation, la table de traitement T est configurée pour déplacer le sujet par rapport au point d'incidence du faisceau, notamment pendant le traitement d'hadronthérapie. Dans ce premier mode de réalisation illustré en Figure 1, la table de traitement T est apte à tourner autour des trois axes A1, A2 et A3 dans les deux sens de rotation et à translater selon les axes A1, A2 et A3 dans les deux directions opposées de chaque axe.

Dans ce premier mode de réalisation, le télescope Compton TC comprend deux semi-détecteurs Compton sous la forme de deux demi-cylindres creux, symétriques par rapport à l'axe A1 et séparés l'un de l'autre selon la direction A2 de manière à permettre le passage d'un faisceau F arrivant au-dessus du sujet. Les deux demi cylindres sont complémentaires et aptes à former un cylindre creux complet si réunis. Notamment, chaque semi-détecteur forme un arc de cercle d'angle 180°. Dans ce mode de réalisation, chaque semi-détecteurs Compton est apte à translater selon les axes A1, A2 et A3 dans les deux directions et tourner autour de l'axe A3.

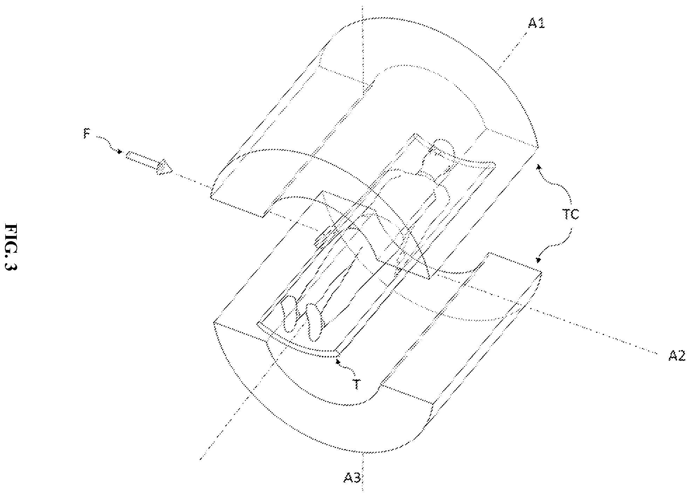

Dans le deuxième mode de réalisation illustré en Figure 3, la table de traitement T est aussi apte à tourner autour des trois axes A1, A2 et A3 dans les deux sens de rotation et à translater selon les axes A1, A2 et A3 dans les deux directions opposées de chaque axe. Le télescope Compton comprend deux semi-détecteurs Compton sous la forme de deux demi-cylindres creux symétriques par rapport à l'axe A1 et séparé l'un de l'autre selon la direction A3 de manière à permettre le passage d'un faisceau F arrivant sur le côté du sujet.

La configuration du télescope Compton dans les premier et deuxième modes de réalisation est appelée dans la présente description « télescope Compton en configuration ouverte ». Dans les premier et deuxième modes de réalisation, chaque semi-détecteurs Compton peut être déplacé indépendamment de l'autre pour s'adapter davantage à la position et direction d'incidence du faisceau F sur le sujet tout en gardant une configuration du télescope Compton TC la plus refermée possible sur le sujet. En effet, le télescope Compton présente une résolution spatiale plus élevée quand les deux semi-détecteurs sont les plus rapprochés du sujet permettant la détection d'un plus grand nombre d'évènements (i.e. prompts gamma, rayon gamma de désexcitation ou gamma d'annihilation).

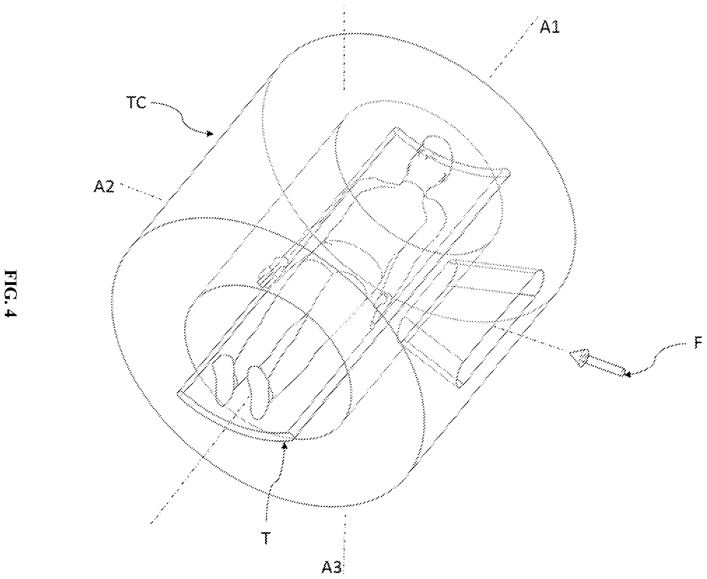

Dans le troisième mode de réalisation illustré en Figure 4, le télescope Compton comprend un seul volume de détection ayant la forme d'un cylindre creux et comprenant une fente latérale ayant des dimensions aptes à permettre le passage d'un faisceau F arrivant sur le côté du sujet. Cette configuration du télescope Compton est appelée dans la présente description « télescope Compton en configuration fermée ». La table de traitement T alignée le long de l'axe A1 étant aussi l'axe longitudinal d'un cylindre creux. La table de traitement T est apte à être déplacée à l'intérieur du télescope Compton en configuration fermée et notamment à tourner autour des trois axes A1, A2 et A3 dans les deux sens de rotation et à translater selon les axes A1, A2 et A3 dans les deux directions opposées de chaque axe. Dans ce mode de réalisation, le télescope Compton est monté sur un support mécanique, motorisé de manière à pouvoir translater selon les axes A1, A2 et A3 et à pivoter autour de l'axe A3.

Dans les premier, deuxième et troisième modes de réalisation, la table de traitement T sur laquelle est positionné le sujet est montée solidement à un support mécanique motorisé (non représenté) qui lui permet de se mouvoir avec les degrés de liberté décrit ci-dessus. Le support mécanique motorisé est notamment apte à permettre des mouvements de rotation de la table de traitement T avec un pas qui varie entre 2 et 15 degrés et des mouvements de translation d'un pas qui varie entre 0.5 cm et 5cm.

Dans un mode de réalisation, le support motorisé est un bras robotique de positionnement configuré pour avoir une sensibilité comprise entre 0.1 cm et 1 cm.

Dans les modes de réalisation comprenant une table de traitement T sur un support mécanique motorisé, au moins un des paramètres prédéfinis du plan de traitement correspond à la position dans l'espace de la table de traitement T et donc du support mécanique motorisé. Ce mode de réalisation, particulièrement avantageux, permet de s'affranchir de l'utilisation de gigantesque têtes rotatives iso-centrique (« Gantry ») configurées pour délivrer le faisceau avec un angle variable sur plus de 180°. Le « Gantry », dans lequel est incorporé le nez de traitement, a un coût de fabrication extrêmement élevé par rapport à celui d'un lit de traitement motorisé. En conséquence, s'affranchir de la « Gantry » permet de réduire le coût de construction des infrastructures d'hadronthérapie rendant cette technique plus accessible.

Dans les modes de réalisation dans lesquels le télescope Compton est apte à être déplacé, au moins un des paramètres prédéfinis du plan de traitement correspond à la position dans l'espace du télescope Compton en configuration fermée ou de chaque semi-détecteur Compton du télescope Compton en configuration ouverte.

Le dispositif d'accélération utilisé dans le procédé de la présente invention produit un faisceau d'hadrons comprenant une pluralité de « burst »s discrets d'hadrons émis à une fréquence prédéfinie par un dispositif d'accélération.

Dans un mode de réalisation, le dispositif d'accélération qui génère le faisceau d'hadron utilisé, est un synchrotron ou un accélérateur linéaire. Les synchrotrons accélèrent les hadrons à l'énergie désirée en paquets (impulsions). Une fois qu'un paquet a atteint l'énergie requise, il est extrait et transmis via la « ligne de faisceau » à la salle de traitement. Toutefois, quelques soit le type d'accélérateur, le faisceau mono-énergétique étroit extrait est guidé magnétiquement à travers la ligne de faisceau jusqu'au nez de traitement.

Dans un mode de réalisation, le dispositif d'accélération est un synchrotron « digital » comprenant des cellules d'induction dans lequel les aimants sont exposés à des variations rapides de leur fonctionnement grâce à des moyens externes de contrôle du faisceau et à des calculateurs embarqués à même de les contrôler (switch et FPGA).

L'interaction d'un « burst » produit dans la matière, en plus de l'auto-activation et de l'émission de particules chargées, l'émission rapide de rayons gamma d'haute énergie appelé « gamma prompts ». Le profil de génération de « gamma prompts » est bien corrélé au parcours des hadrons. En conséquence, la détection de « gamma prompts » peut être efficacement utilisée pour suivre le point d'interaction du faisceau et le dépôt de dose dans le sujet.

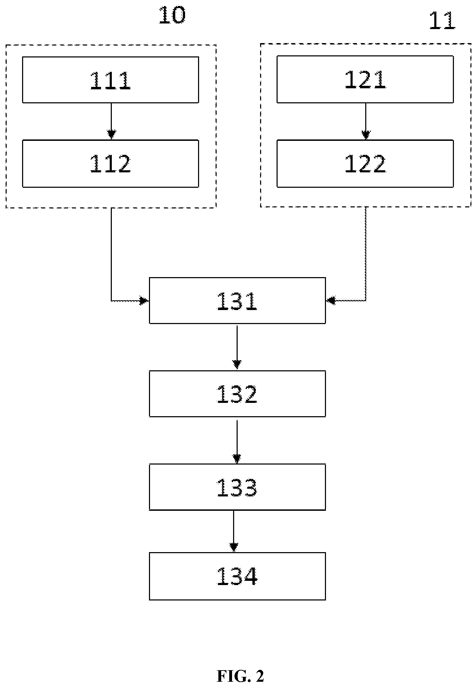

En Figure 2 est illustré un mode de réalisation du procédé comprenant plusieurs étapes d'acquisition des données et de calculs.

Le procédé décrit à titre illustratif est configuré pour permettre le suivi en temps réel d'un traitement d'hadronthérapie en obtenant des séquences d'images du volume d'interaction à l'intérieur duquel les hadrons du « burst » interagissent avec les tissus du sujet et du volume total de la tumeur. L'image du volume d'interaction peut être obtenue seulement pendant qu'un « burst » impact le sujet.

Le procédé comprend donc une première phase, quand un « burst » impacte le sujet 10, cette première phase comprenant les étapes suivantes :
- détecter les « gamma prompts »111 générés par l'interaction des hadrons du « burst » avec les tissus du sujet à l'aide d'un télescope Compton;
- utiliser les « gamma prompts » détectés pour reconstruire une image 112 du volume d'interaction à l'intérieur duquel les hadrons du « burst » interagissent avec les tissus du sujet.

Dans un mode de réalisation préféré, le télescope Compton est un télescope Compton à Xénon liquide. Les propriétés physiques fondamentales du Xénon liquide, tels quel sa haute densité et son numéro atomique élevé, confèrent un pouvoir d'arrêt élevé aux rayonnements ionisants, ce qui fait du Xénon liquide un matériau idéal en tant que détecteur de rayons gamma dans la gamme d'énergie allant de quelques dizaines de keV à plusieurs dizaines de MeV. Le Xénon liquide est à la fois un excellent milieu actif pour la détection de rayonnements ionisants et un excellent scintillateur, avec l'avantage de rendre possible la construction de détecteurs de grandes tailles avec un milieu sensible homogène. Un télescope Compton utilise les interactions successives (deux ou plus) d'un photon incident dans le volume d'interaction. A partir des points d'interactions et de l'énergie déposée à chaque interaction, la direction du photon incident peut être réduit à un cône, via l'application de la cinématique Compton. La position de la source de photon peut être déterminée par l'intersection de différents cônes Compton, déduit des interactions ultérieures des photons provenant de la même source. Ce type de détecteur est donc bien adapté pour détecter des « gamma prompts » dans la plage d'énergie correspondante de plusieurs MeV dans laquelle la diffusion Compton est le process dominant.

L'image du volume d'interaction mesuré peut être une image tridimensionnelle.

Dans un mode de réalisation, l'image du volume d'interaction est obtenue pour chaque « burst » impactant le sujet. Dans ce mode de réalisation, la fréquence d'acquisition des images dépend de la fréquence de production de « burst »s par le dispositif d' accélération. Le numéro d'image acquis par second peut donc varier de 5 à 30.

Dans un mode de réalisation, le télescope Compton est configuré pour avoir une résolution spatiale d'approximativement 1mm pour chaque image d'un « burst ».

Dans la présente invention, les cellules tumorales du sujet sont marquées avec un produit radiopharmaceutique comprenant un radio-isotope émettant un positron et un rayon gamma de désexcitation en quasi-coïncidence. Cela permet l'identification de la position du radio-marqueur grâce à l'utilisation du principe d'imagerie à trois photons. En effet, le positron parcourt un bref trajet dans les tissus (de l'ordres du mm) avant d'annihiler en deux photons à 511 keV ses déplaçant en direction opposée (« back-to-back gammas »).

L'imagerie à 3 photons est basée sur l'utilisation conjointe d'un télescope Compton et d'un radiopharmaceutique ayant un radio-isotope émettant un positron et un rayon gamma de désexcitation en quasi-coïncidence.

La détection simultanée de ces deux photons d'annihilation permet de tracer la ligne de réponse (LOR), c'est à dire une ligne connectant les deux points d'interactions des photons d'annihilation dans le télescope Compton et passant par le point d'annihilation du positron dans le tissu. La position du radio-isotope est donc obtenue par l'intersection entre LOR et le cône Compton défini à partir de l'interaction du gamma de désexcitation avec le télescope Compton. La surface du cône Compton comprend la direction incidente du gamma de désexcitation et peut être directement déduite de la cinématique Compton. L'angle d'ouverture du cône Compton, θ, est défini par la formule de diffusion Compton, dans laquelle l'axe du cône Compton est déterminé en utilisant le premier des deux points d'interaction du gamma de désexcitation entrant dans le télescope.

Cette technique permet avantageusement une réduction du nombre de désintégrations nécessaires pour l'obtention de l'image et donc la réduction du temps d'acquisition et/ou de la quantité de substance inoculée par patient.

La première phase du procédé comprend, quand aucun « burst » n'impacte le sujet 11, les étapes suivantes :
- extraire la position des cellules tumorales 121 marquées avec le produit radiopharmaceutique en détectant simultanément le rayon gamma de désexcitation et deux rayons gamma d'annihilation produits par le positron à l'aide du télescope Compton, comme décrit ci-dessus ;
- reconstruire, sur la base de positions des cellules tumorales, une image du volume total de la tumeur 122 à traiter par le faisceau d'hadrons pendant le traitement d'hadronthérapie.

Dans un mode de réalisation préféré, le radio-isotope utilisé dans le radiopharmaceutique est le ⁴⁴Sc, qui émet un positon (ou « positron » en Anglais) et un photon d'énergie 1,157 MeV en quasi-coïncidence spatiale et temporelle.

L'image du volume total de la tumeur est acquise entre deux « burst »s successifs pour éviter une contamination de l'image par le signal généré par les « gamma prompts ».

Dans un exemple, la structure du télescope Compton est telle que pour une acquisition de 10 s la résolution spatiale de l'image du volume total de la tumeur est d'environ 1 mm. La résolution atteint est due aux limites physiques du détecteur et à l'activité de l'isotope injecté dans le sujet.

Dans un mode de réalisation, l'image du volume total de la tumeur est une image tridimensionnelle.

Le procédé comprend en outre une deuxième phase comprenant les étapes suivantes :
- comparer l'image du volume d'interaction et l'image du volume total de la tumeur 131 de sorte à localiser le volume d'interaction mesuré par rapport au volume total mesuré de la tumeur;
- à chaque fois que le volume d'interaction mesuré est compris au moins partiellement dans le volume total mesuré de la tumeur, calculer l'écart 132 entre, d'une part, la position du volume d'interaction mesuré dans le volume total mesuré de la tumeur et, d'autre part, une position prédéfinie du volume d'interaction dans le volume total de la tumeur définie dans le plan de traitement.

L'écart peut être calculé par superposition des images. Ceci est facile à obtenir, du moment que les images du volume d'interaction mesuré et du le volume total de la tumeur mesuré sont acquis par le même détecteur et sont donc reconstruite par rapport à un même référentiel.

Cette phase de comparaison permet de comparer la position réelle du pic de Bragg par rapport à la position qui a été calculée dans un logiciel de calcul pour la génération du plan de traitement.

Dans un mode de réalisation, le procédé comprend en outre une étape 133 dans laquelle l'écart entre la position prédéfinie du volume d'interaction et la position du volume d'interaction mesuré par rapport au volume total mesuré de la tumeur est comparé à un seuil prédéfini. Le seul prédéfini peut correspondre à une marge d' 1 mm à 5 mm autour du volume cible (PTV). Le seuil peut aussi être défini sur la base de la radiosensibilité des organes à risque qui entoure le volume ciblé. Pour exemple, le seuil prédéfini correspond à une marge de 0.5 mm dans la direction des ganglions qui sont hautement radiosensible et une marge de 1mm dans la direction des poumons qui le sont moins.

Dans un mode de réalisation, à chaque fois que l'écart est supérieur au seuil prédéfini, le procédé comprend une étape de calcul d'au moins un nouveau paramètre du faisceau d'hadrons 134 de manière à corriger au moins une caractéristique du faisceau d'hadrons.

Dans un mode de réalisation, à chaque fois que l'écart est supérieur au seuil prédéfini, le procédé comprend une étape de calcul du paramètre du plan de traitement correspondant à une nouvelle position dans l'espace du support mécanique motorisé de manière à corriger au moins une caractéristique du faisceau d'hadrons.

Dans un mode de réalisation, le nouveau paramètre du faisceau d'hadrons est transmis au dispositif d'accélération qui modifie au moins une caractéristique du faisceau d'hadrons de manière à modifier la position du volume d'interaction par rapport au volume total mesuré de la tumeur.

Dans un mode de réalisation, à chaque fois que l'écart est supérieur au seuil prédéfini, le procédé comprend l'arrêt du faisceau d'hadrons. Cela permet d'avoir une mesure de sécurité pour éviter tout irradiation non conforme au plan de traitement.

Dans un mode de réalisation, le procédé comprend en outre une étape de reconstruction d'une séquence d'image tridimensionnelle résultant de la fusion d'une image tridimensionnelle du volume d'interaction avec une image tridimensionnelle du volume total de la tumeur selon un même référentiel de la caméra Compton. Une telle séquence d'images peut être visualisée en temps réel grâce à un écran pour donner une information visuelle au corps médical.

L'invention a pour objet un système de suivi d'un faisceau d'hadrons pendant un traitement d'hadronthérapie d'un sujet. Le système est configuré pour suivre un faisceau d'hadron, comprenant une pluralité de « burst »s discrets d'hadrons émis à une fréquence prédéfinie par un dispositif d'accélération, pendant un traitement d'hadronthérapie délivré selon un plan de traitement comprenant des paramètres prédéfinis en fonction du temps afin de définir au moins une caractéristique du faisceau d'hadrons dans le temps pendant le traitement d'hadronthérapie.

Le système de suivi d'un faisceau d'hadrons va maintenant être détaillé. Dans ce qui suit, les modules doivent être compris comme des entités fonctionnelles plutôt que comme des composants matériels, physiquement distincts. Ils peuvent donc être soit regroupés dans un même composant matériel et concret, soit répartis en plusieurs de ces composants. En outre, chacun de ces modules est éventuellement lui-même partagé entre au moins deux composantes physiques. En outre, les modules sont implémentés dans du matériel, des logiciels, des microprogrammes ou toute autre forme mixte de ceux-ci.

Le système comprend un module d'imagerie de faisceau configuré pour recevoir les données acquises d'un télescope Compton lorsqu'un « burst » impacte le sujet. Dans un mode de réalisation, l'acquisition de données est effectuée on-line et en continue. Les données acquises pendant une fenêtre temporale, correspondant à l'envoie d'un « burst » sur le sujet, sont envoyées au module d'imagerie de faisceau. Ce module est en outre configuré pour analyser ces données de manière à déterminer le point d'émission des « gamma prompts » généré par l'interaction du « burst » avec les tissus du sujet. Ce module utilise donc le point d'émission des « gamma prompts » pour reconstruire une image du volume d'interaction à l'intérieur duquel les hadrons du « burst » interagissent avec les tissus.

Dans un mode de réalisation, le module d'imagerie de faisceau est en outre configuré pour identifier et rééjecter toutes données correspondant à des évènements qui ne sont pas issus de l'interaction d'un « gamma prompts » dans le volume sensible du télescope Compton. Le système comprend aussi un module d'imagerie tumorale configuré pour recevoir les données acquises par le télescope Compton lorsqu'aucun « burst » n'impacte le sujet.

Dans un mode de réalisation, l'acquisition de données est effectuée on-line et en continue et seulement les données acquises entre deux « burst »s successifs sujet sont envoyés au module d'imagerie tumorale. Ceci permet de sélectionner seulement les données issues de la désintégration beta du radio-isotope captée par les cellules tumorales. Ce module est en outre configuré pour analyser ces données de manière à extraire la position des cellules tumorales marquées avec le produit radiopharmaceutique en détectant simultanément le rayon gamma de désexcitation et les deux rayons gamma d'annihilation produit par le positron et utiliser la position des cellules tumorales pour reconstruire une image du volume total de la tumeur à traiter par le faisceau d'hadrons pendant le traitement d'hadronthérapie.

Dans un mode de réalisation, le télescope Compton est un télescope Compton à Xénon liquide.

Le système comprend en outre un module d'évaluation configuré pour comparer l'image du volume d'interaction et l'image du volume total de la tumeur de sorte à localiser le volume d'interaction mesuré par rapport au volume total mesuré de la tumeur et, à chaque fois que le volume d'interaction mesuré est compris, au moins partiellement, dans le volume total mesuré de la tumeur pour calculer l'écart entre, d'une part, la position du volume d'interaction mesuré par rapport au volume total mesuré de la tumeur et, d'autre part, une position prédéfinie du volume d'interaction au sein du volume total de la tumeur définie dans le plan de traitement.

Dans un mode de réalisation, le module d'évaluation est en outre configuré pour comparer l'écart entre la position prédéfinie du volume d'interaction et la position du volume d'interaction mesuré par rapport au volume total mesuré de la tumeur, à un seuil prédéfini.

Dans un mode de réalisation, le système comprend en outre un module de correction configuré pour calculer au moins un nouveau paramètre du faisceau d'hadrons à chaque fois que l'écart est supérieur au seuil prédéfini, afin de corriger au moins une caractéristique du faisceau d'hadrons.

Comme expliqué dans la première partie de la description, les paramètres prédéfinis définissant les caractéristiques du faisceau d'hadrons varient selon le type de traitement de hadronthérapie. Sur la base du type de traitement qui peut être délivré par le nez de traitement, les paramètres prédéfinis pouvant être influencés dynamiquement pendant une séance comprennent l'énergie du faisceau, la modulation du trajet des hadrons (i.e. SOBP), la modulation du trajet des hadrons et/ou la position en X et Y du faisceau.

Dans un mode de réalisation, le module de correction est en outre configuré pour transmettre le nouveau paramètre du faisceau d'hadrons au dispositif d'accélération qui modifie au moins une caractéristique du faisceau d'hadrons de manière à modifier la position du volume d'interaction par rapport au volume total mesuré de la tumeur.

Dans un mode de réalisation, le dispositif d'accélération comprend un nez de traitement disposé dans la sale de traitement en position fixe par au table de traitement.

Dans un mode de réalisation, la table de traitement T est configurée pour déplacer le sujet par rapport au point d'incidence du faisceau, notamment pendant le traitement d'hadronthérapie. Dans ce premier mode de réalisation illustré en Figure 1, la table de traitement T est apte à tourner autour des trois axes A1, A2 et A3 dans les deux sens de rotation et à translater selon les axes A1, A2 et A3 dans les deux directions opposées de chaque axe.

Dans ce premier mode de réalisation, le télescope Compton TC comprend deux semi-détecteurs Compton sous la forme de deux demi-cylindres creux, symétriques par rapport à l'axe A1 et séparés l'un de l'autre selon la direction A2 de manière à permettre le passage d'un faisceau F arrivant au-dessus du sujet. Les deux demi cylindres sont complémentaires et aptes à former un cylindre creux complet si réunis. Notamment, chaque semi-détecteur forme un arc de cercle d'angle 180°. Dans ce mode de réalisation, chaque semi-détecteurs Compton est apte à translater selon les axes A1, A2 et A3 dans les deux directions et tourner autour de l'axe A3.

Dans le deuxième mode de réalisation illustré en Figure 3, la table de traitement T est aussi apte à tourner autour des trois axes A1, A2 et A3 dans les deux sens de rotation et à translater selon les axes A1, A2 et A3 dans les deux directions opposées de chaque axe. Le télescope Compton comprend deux semi-détecteurs Compton sous la forme de deux demi-cylindres creux symétriques par rapport à l'axe A1 et séparé l'un de l'autre selon la direction A3 de manière à permettre le passage d'un faisceau F arrivant sur le côté du sujet.

La configuration du télescope Compton dans les premier et deuxième modes de réalisation est appelée dans la présente description « télescope Compton en configuration ouverte ». Dans les premier et deuxième modes de réalisation, chaque semi-détecteurs Compton peut être déplacé indépendamment de l'autre pour s'adapter davantage à la position et direction d'incidence du faisceau F sur le sujet tout en gardant une configuration du télescope Compton TC la plus refermée possible sur le sujet. En effet, le télescope Compton présente une résolution spatiale plus élevée quand les deux semi-détecteurs sont les plus rapprochés du sujet permettant la détection d'un plus grand nombre d'évènements (i.e. prompts gamma, rayon gamma de désexcitation ou gamma d'annihilation).

Dans le troisième mode de réalisation illustré en Figure 4, le télescope Compton comprend un seul volume de détection ayant la forme d'un cylindre creux et comprenant une fente latérale ayant des dimensions aptes à permettre le passage d'un faisceau F arrivant sur le côté du sujet. Cette configuration du télescope Compton est appelée dans la présente description « télescope Compton en configuration fermée ». La table de traitement T alignée le long de l'axe A1, étant aussi l'axe longitudinal d'un cylindre creux. La table de traitement T est apte à être déplacée à l'intérieur du télescope Compton en configuration fermée et notamment à tourner autour des trois axes A1, A2 et A3 dans les deux sens de rotation et à translater selon les axes A1, A2 et A3 dans les deux directions opposées de chaque axe. Dans ce mode de réalisation, le télescope Compton est monté sur un support mécanique, motorisé de manière à pouvoir translater selon les axes A1, A2 et A3 et à pivoter autour de l'axe A3.

Dans les premier, deuxième et troisième modes de réalisation, la table de traitement T sur laquelle est positionné le sujet est montée solidement à un support mécanique motorisé (non représenté) qui lui permet de se mouvoir avec les degrés de liberté décrit ci-dessus. Le support mécanique motorisé est notamment apte à permettre des mouvements de rotation de la table de traitement T avec un pas qui varie entre 2 et 15 degrés et des mouvements de translation d'un pas qui varie entre 0.5 cm et 5cm.

Dans un mode de réalisation, le support motorisé est un bras robotique de positionnement configuré pour avoir une sensibilité comprise entre 0.1 cm et 1 cm.

Dans les modes de réalisation comprenant une table de traitement T sur un support mécanique motorisé, au moins un des paramètres prédéfinis du plan de traitement correspond à la position dans l'espace du support mécanique motorisé, notamment la table de traitement T. Le module de correction est en outre configuré pour calculer un nouveau paramètre du plan de traitement correspondant à une nouvelle position dans l'espace du support mécanique motorisé de manière à corriger au moins une caractéristique du faisceau d'hadrons, correspondant notamment à la direction et le point d'incidence du faisceau.

Dans les modes de réalisation dans lesquels le télescope Compton est apte à être déplacé, au moins un des paramètres prédéfinis du plan de traitement correspond à la position dans l'espace du télescope Compton en configuration fermée ou de chaque semi-détecteur Compton du télescope Compton en configuration ouverte. Le module de correction est en outre configuré pour calculer un nouveau paramètre du plan de traitement correspondant à une nouvelle position dans l'espace du télescope Compton en configuration fermée ou de chaque semi-détecteur Compton du télescope Compton en configuration ouverte.

Dans un mode de réalisation, le module de correction est en outre configuré pour transmettre le nouveau paramètre du faisceau d'hadrons concernant une nouvelle position du support mécanique motorisé au support mécanique motorisé, lui-même de sorte à modifier la direction et/ou le point d'incidence du faisceau et donc la position du volume d'interaction par rapport au volume total mesuré de la tumeur.

Dans un mode de réalisation, le système comprend un module de reconstruction d'image configuré pour reconstruire une séquence d'images tridimensionnelles résultant de la fusion d'une image tridimensionnelle du volume d'interaction avec une image tridimensionnelle du volume total de la tumeur selon un même référentiel de la caméra Compton. Ceci permet d'obtenir un film de la séance de traitement.

Dans un mode de réalisation, les modules du système comprennent au moins un processeur et au moins un support d'enregistrement lisible par processeur.

Le terme « processeur » ne doit pas être interprété comme étant limité à en « hardware » capable d'exécuter un logiciel, mais désigne de manière générale un dispositif de traitement, qui peut par exemple inclure un ordinateur, un microprocesseur, un circuit intégré ou un dispositif logique programmable (PLD). Le processeur peut également comprendre une ou plusieurs unités de traitement graphique (GPU), qu'elles soient exploitées pour l'infographie et le traitement d'images ou pour d'autres fonctions. En outre, les instructions et/ou les données permettant d'exécuter les fonctionnalités associées et/ou résultantes peuvent être stockées sur tout support lisible par un processeur comme, par exemple, un circuit intégré, un disque dur, un CD (Compact Disc), un disque optique tel qu'un DVD (Digital Versatile Disc), une RAM (Random-Access Memory) ou une ROM (Read-Only Memory). Les instructions peuvent notamment être stockées dans du matériel, des logiciels, des microprogrammes ou dans toute combinaison de ceux-ci.

La présente divulgation a également pour objet un programme d'ordinateur comprenant des instructions qui, lorsque le programme est exécuté par un ordinateur, conduisent celui-ci à mettre en œuvre le procédé de suivi d'un faisceau d'hadrons décrit ci-dessus.

La présente divulgation a également pour objet un support d'enregistrement lisible par ordinateur comprenant des instructions qui, lorsqu'elles sont exécutées par un ordinateur, conduisent celui-ci à mettre en œuvre le procédé de suivi d'un faisceau d'hadrons décrit ci-dessus. Dans un mode de réalisation, le support d'enregistrement lisible par ordinateur est non transitoire.

## Revendications

1. Système de suivi d'un faisceau d'hadrons pendant un traitement d'hadronthérapie d'un sujet comprenant des cellules tumorales marquées avec un produit radiopharmaceutique ayant un radio-isotope émettant un positron et un rayon gamma de désexcitation en quasi-coïncidence, dans lequel le traitement d'hadronthérapie est délivré selon un plan de traitement comprenant des paramètres prédéfinis en fonction du temps afin de définir au moins une caractéristique du faisceau d'hadrons dans le temps pendant le traitement d'hadronthérapie, dans lequel le faisceau d'hadrons comprend une pluralité de bursts discrets d'hadrons émis à une fréquence prédéfinie par un dispositif d'accélération, le système comprenant :
- un module d'imagerie de faisceau configuré pour recevoir les données acquises d'un télescope Compton à xénon liquide lorsqu'un burst impacte le sujet et pour analyser ces données de manière à déterminer le point d'émission des gamma prompts générés par l'interaction du burst avec les tissus du sujet à partir duquel est reconstruite une image du volume d'interaction à l'intérieur duquel les hadrons du burst interagissent avec les tissus ;
- un module d'imagerie tumorale configuré pour recevoir les données acquises par le télescope Compton, notamment un télescope Compton à xénon liquide, lorsqu'aucun burst n'impacte le sujet et pour analyser ces données de manière à extraire la position des cellules tumorales marquées avec le produit radiopharmaceutique en détectant simultanément le rayon gamma de désexcitation et deux rayons gamma d'annihilation produits par le positron et utiliser la position des cellules tumorales pour reconstruire une image du volume total de la tumeur à traiter par le faisceau d'hadrons pendant le traitement d'hadronthérapie ;
- un module d'évaluation configuré pour comparer l'image du volume d'interaction et l'image du volume total de la tumeur de sorte à localiser le volume d'interaction mesuré par rapport au volume total mesuré de la tumeur et, à chaque fois que le volume d'interaction mesuré est compris au moins partiellement dans le volume total mesuré de la tumeur, pour calculer l'écart entre, d'une part, la position du volume d'interaction mesuré par rapport au volume total mesuré de la tumeur et, d'autre part, une position prédéfinie du volume d'interaction au sein du volume total de la tumeur définie dans le plan de traitement.

2. Système selon la revendication 1, dans lequel le module d'évaluation est en outre configuré pour comparer l'écart entre la position prédéfinie du volume d'interaction et la position du volume d'interaction mesuré par rapport au volume total mesuré de la tumeur, à un seuil prédéfini.

3. Système selon l'une quelconque des revendications 1 ou 2, comprenant en outre un module de correction configuré pour calculer au moins un nouveau paramètre du faisceau d'hadrons, à chaque fois que l'écart est supérieur au seuil prédéfini, afin de corriger au moins une caractéristique du faisceau d 'hadrons.

4. Système selon la revendication 3, dans lequel le module de correction est en outre configuré pour transmettre le nouveau paramètre du faisceau d'hadrons au dispositif d'accélération, qui modifie au moins une caractéristique du faisceau d'hadrons de manière à modifier la position du volume d'interaction par rapport au volume total mesuré de la tumeur.

5. Système selon l'une quelconque des revendications 1 à 4, comprenant en outre un module de sécurité configuré pour transmettre au dispositif d'accélération l'instruction d'arrêter le faisceau d'hadrons à chaque fois que l'écart est supérieur au seuil prédéfini.

## Patentansprüche

1. System zur Überwachung eines Hadron-Jets während einer Hadrontherapiebehandlung einer Person, die Tumorzellen aufweist, die mit einem Radiopharmazeutikum markiert sind, das ein Radioisotop besitzt, das ein Positron und einen Entregungsgammastrahl in Quasi-Koinzidenz emittiert, wobei die Hadrontherapiebehandlung nach einem Behandlungsplan verabreicht wird, der in Abhängigkeit von der Zeit vorgegebene Parameter aufweist, um mindestens ein Merkmal des Hadron-Jets in der Zeit während der Hadrontherapiebehandlung zu definieren, wobei der Hadron-Jet eine Vielzahl von diskreten Hadron-Bursts umfasst, die mit einer vorgegebenen Frequenz von einer Beschleunigungsvorrichtung emittiert werden, wobei das System umfasst:
- ein Jetbildgebungsmodul, das dazu ausgestaltet ist, Daten zu empfangen, die von einem Flüssig-Xenon-Compton-Teleskop erfasst werden, wenn ein Burst auf die Person trifft, und diese Daten so zu analysieren, dass der Emissionspunkt der durch die Wechselwirkung des Bursts mit den Geweben der Person erzeugten Prompt-Gammas bestimmt wird, ab dem ein Bild des Interaktionsvolumens rekonstruiert wird, in dem die Hadronen des Bursts mit den Geweben interagieren;
- ein Tumorbildgebungsmodul, das dazu ausgestaltet ist, die Daten zu empfangen, die von dem Compton-Teleskop, insbesondere einem Flüssig-Xenon-Compton-Teleskop, erfasst werden, wenn kein Burst auf die Person trifft, und diese Daten so zu analysieren, dass die Position der mit dem Radiopharmazeutikum markierten Tumorzellen extrahiert wird, indem gleichzeitig der Entregungsgammastrahl und zwei durch das Positron erzeugte Annihilationsgammastrahlen detektiert werden, und die Position der Tumorzellen zu verwenden, um ein Bild des Gesamtvolumens des mit dem Hadron-Jet während der Hadrontherapiebehandlung zu behandelnden Tumors zu rekonstruieren;
- ein Evaluierungsmodul, das dazu ausgestaltet ist, das Bild des Interaktionsvolumens und das Bild des Gesamtvolumens des Tumors zu vergleichen, so dass das gemessene Interaktionsvolumen in Bezug auf das gemessene Gesamtvolumen des Tumors lokalisiert wird, und jedes Mal dann, wenn das gemessene Interaktionsvolumen mindestens teilweise in dem gemessenen Gesamtvolumen des Tumors enthalten ist, die Differenz zu berechnen zwischen der Position des gemessenen Interaktionsvolumens in Bezug auf das gemessene Gesamtvolumen des Tumors zum einen und einer vorgegebenen Position des Interaktionsvolumens innerhalb des Gesamtvolumens des Tumors, die in dem Behandlungsplan definiert ist, zum anderen.

2. System nach Anspruch 1, wobei das Evaluierungsmodul ferner dazu ausgestaltet ist, die Differenz zwischen der vorgegebenen Position des Interaktionsvolumens und der Position des gemessenen Interaktionsvolumens in Bezug auf das gemessene Gesamtvolumen des Tumors mit einem vorgegebenen Schwellenwert zu vergleichen.

3. System nach einem der Ansprüche 1 oder 2, umfassend ferner ein Korrekturmodul, das dazu ausgestaltet ist, mindestens einen neuen Parameter des Hadron-Jets jedes Mal dann zu berechnen, wenn die Differenz größer als der vorgegebene Schwellenwert ist, um mindestens ein Merkmal des Hadron-Jets zu korrigieren.

4. System nach Anspruch 3, wobei das Korrekturmodul ferner dazu ausgestaltet ist, den neuen Parameter des Hadron-Jets an die Beschleunigungsvorrichtung zu übertragen, die mindestens ein Merkmal des Hadron-Jets ändert, so dass die Position des Interaktionsvolumens in Bezug auf das gemessene Gesamtvolumen des Tumors geändert wird.

5. System nach einem der Ansprüche 1 bis 4, umfassend ferner ein Sicherheitsmodul, das dazu ausgestaltet ist, an die Beschleunigungsvorrichtung jedes Mal dann die Anweisung zu übertragen, den Hadron-Jet zu stoppen, wenn die Differenz größer als der vorgegebene Schwellenwert ist.

## Claims

1. System for monitoring a hadron beam during particle therapy treatment of a subject comprising tumour cells labelled with a radiopharmaceutical product having a radio-isotope quasi-coincidentally emitting a positron and a de-excitation gamma ray, wherein the particle therapy treatment is delivered in accordance with a treatment plan comprising parameters that are predefined as a function of time so as to define at least one characteristic of the hadron beam over time during the particle therapy treatment, wherein the hadron beam comprises a plurality of discrete hadron bursts emitted at a predefined frequency by an acceleration device, the system comprising:
- a beam imaging module configured to receive data acquired from a liquid-xenon Compton telescope when a burst impacts the subject and to analyse these data so as to determine the point of emission of prompt gammas generated by the interaction of the burst with the tissue of the subject, which is taken as a basis for reconstructing an image of the interaction volume within which the hadrons of the burst interact with the tissue;
- a tumour imaging module configured to receive the data acquired by the Compton telescope, in particular a liquid-xenon Compton telescope, when no burst impacts the subject, and to analyse these data so as to extract the position of the tumour cells labelled with the radiopharmaceutical product by simultaneously detecting the de-excitation gamma ray and two annihilation gamma rays produced by the positron and to use the position of the tumour cells to reconstruct an image of the total volume of the tumour to be treated by the hadron beam during the particle therapy treatment;
- an evaluation module configured to compare the image of the interaction volume and the image of the total volume of the tumour so as to locate the measured interaction volume with respect to the measured total volume of the tumour and, whenever the measured interaction volume is at least partially contained within the measured total volume of the tumour, to calculate the deviation between the position of the measured interaction volume with respect to the measured total volume of the tumour, on the one hand, and a predefined position of the interaction volume within the total volume of the tumour defined in the treatment plan, on the other hand.

2. System according to Claim 1, wherein the evaluation module is furthermore configured to compare the deviation between the predefined position of the interaction volume and the position of the measured interaction volume with respect to the measured total volume of the tumour with a predefined threshold.

3. System according to either one of Claims 1 and 2, furthermore comprising a correction module configured to calculate at least one new parameter of the hadron beam whenever the deviation is greater than the predefined threshold, in order to correct at least one characteristic of the hadron beam.

4. System according to Claim 3, wherein the correction module is furthermore configured to transmit the new parameter of the hadron beam to the acceleration device, which modifies at least one characteristic of the hadron beam so as to modify the position of the interaction volume with respect to the measured total volume of the tumour.

5. System according to any one of Claims 1 to 4, furthermore comprising a safety module configured to transmit, to the acceleration device, the instruction to stop the hadron beam whenever the deviation is greater than the predefined threshold.
